# EUROPÄISCHE PATENTSCHRIFT

(11) **EP 1 215 194 B1**
(45) Veröffentlichungstag und Bekanntmachung des Hinweises auf die Patenterteilung: **27.08.2008**
(21) Anmeldenummer: 01127326.5
(22) Anmeldetag: 20.11.2001
(51) Int. Cl.: C07C 67/08, C07C 69/54

(54) **Verfahren zum Herstellen eines Esters der Acrylsäure oder Methacrylsäure**
Process for the preparation of acrylic or methacrylic acid esters
Procédé de préparation d'esters d'acide acrylique ou méthacrylique

(30) Priorität: 15.12.2000 DE 10062754
(43) Veröffentlichungstag der Anmeldung: 19.06.2002
(73) Patentinhaber: Lurgi GmbH, 60295 Frankfurt am Main (DE)
(72) Erfinder: Buchold, Henning, 63452 Hanau (DE); Dropsch, Holger, 61118 Bad Vilbel (DE); Rothaemel, Martin, 60437 Frankfurt am Main (DE)

(56) Entgegenhaltungen:
- EP-A- 0 594 008
- EP-A- 0 765 859
- WO-A-98/52903
- DE-A- 3 308 879
- GB-A- 1 535 489

## Beschreibung

Die Erfindung betrifft ein Verfahren zur Herstellung eines Esters der Acrylsäure oder Methacrylsäure durch Umsetzung mit einem aliphatischen Alkohol mit 1 bis 10 C-Atomen pro Molekül an einem Katalysator.

Verfahren dieser Art, bei denen der Katalysator in einem einzigen Veresterungsreaktor angeordnet ist, sind bekannt und z.B. in DE-A-19952449, in DE-C-2449811 (hierzu korrespondiert das US-Patent 4012439) und im US-Patent 5645696 beschrieben.

Der Erfindung liegt die Aufgabe zu Grunde, das Verfahren flexibler zu gestalten, so dass es auch für große Anlagen geeignet ist, wobei man bei hoher Produktreinheit kostengünstig arbeiten kann.

Erfindungsgemäß wird die Aufgabe dadurch gelöst, dass man die Umsetzung in mindestens zwei und höchstens fünf in Serie geschalteten Reaktionsstufen durchführt. Jede Reaktionsstufe weist einen Katalysator enthaltenden Veresterungsreaktor und eine Destillationskolonne zum Trennen des vom Reaktor kommenden flüssigen Produkts auf. In jeder Reaktionsstufe wird das von der Destillationskolonne kommende Kopfprodukt gekühlt und kondensiert und aus dem Kondensat wird eine organische, esterhaltige Phase abgetrennt, welche man mindestens teilweise einer destillativen Produktreinigung zuführt. In der ersten bis zweitletzten Reaktionsstufe zieht man jeweils aus dem Sumpf der Destillationskolonne ein Sumpfprodukt ab, welches Ester und Säure enthält, und man leitet dieses Sumpfprodukt mindestens teilweise in den Veresterungsreaktor der folgenden Reaktionsstufe. Acrylsäure oder Methacrylsäure leitet man zusammen mit dem aliphatischen Alkohol und einem Polymerisationsinhibitor in den Veresterungsreaktor der ersten Reaktionsstufe. In diesem ersten Veresterungsreaktor beträgt der Druck 1,5 bis 10 bar. Aus dem Reaktor der ersten Reaktionsstufe zieht man ein Ester, Säure, Alkohol und Wasser enthaltendes erstes flüssiges Gemisch ab, dessen Estergehalt 20 bis 100 % und vorzugsweise 30 bis 80 % des theoretisch möglichen Estergehalts beträgt. In der ersten Reaktionsstufe wird das erste flüssige Gemisch in die Destillationskolonne hinein entspannt, wobei der Druck in dieser Kolonne um mindestens 1 bar niedriger ist als im Veresterungsreaktor. Das aus der Kolonne abgezogene dampfförmige Kopfprodukt enthält Wasserdampf und organische Komponenten, es wird gekühlt und kondensiert und das Kondensat wird in einem zur ersten Reaktionsstufe gehörenden ersten Separator geleitet. Aus dem ersten Separator zieht man eine erste organische Phase und getrennt davon eine erste alkoholhaltige Wasserphase ab. Die Wasserphase wird mindestens teilweise in die erste Destillationskolonne zurückgeführt. Das vom Sumpfbereich der ersten Kolonne kommende Sumpfprodukt enthält Ester, Alkohol und Säure, dieses Sumpfprodukt wird in den Veresterungsreaktor der zweiten Reaktionsstufe geleitet, in welchem der Druck 1,5 bis 10 bar beträgt. Das aus dem zweiten Reaktor abgezogene flüssige Gemisch enthält Ester, Säure, Alkohol und Wasser, es wird in die zweite Destillationskolonne hinein entspannt, wobei der Druck in der zweiten Kolonne um mindestens 1 bar niedriger ist als im zweiten Reaktor. Das aus der zweiten Kolonne kommende zweite Kopfprodukt, welches Wasserdampf und organische Komponenten enthält, wird ebenfalls gekühlt, kondensiert und das Kondensat wird in einen zweiten Separator geleitet, der ebenfalls noch zur zweiten Reaktionsstufe gehört. Aus dem zweiten Separator zieht man eine zweite organische Phase und getrennt davon eine zweite Wasserphase ab. Diese Wasserphase wird mindestens teilweise in die zweite Kolonne zurückgeführt. Mindestens ein Teil des Sumpfprodukts der Kolonne der letzten Reaktionsstufe wird einem Veresterungsreaktor einer vorausgehenden Reaktionsstufe zugeführt. Aus der destillativen Produktreinigung gewinnt man den gewünschten Ester als Produkt. Zumeist sind 2, 3 oder 4 in Serie geschaltete Reaktionsstufen ausreichend, um die gewünschte Produktreinheit zu erzielen.

Vorteilhafterweise zieht man in der letzten Reaktionsstufe aus der zugehörigen Destillationskolonne ein Sumpfprodukt ab und gibt einen Teil dieses Sumpfprodukts der destillativen Produktreinigung auf.

Beim erfindungsgemäßen Verfahren ist es möglich, dass man aus den Destillationskolonnen der ersten und zweiten Reaktionsstufe ein Sumpfprodukt abzieht, welches Acrylsäure oder Methacrylsäure mit einem Anteil von 1 bis 30 Gew.-% enthält, wobei der Wasseranteil bei höchstens 2 Gew.-%, vorzugsweise bei höchstens 1 Gew.-%, liegt. Dieses ist vorteilhaft, weil durch den geringen Wassergehalt für die Umsetzung in der nachfolgenden zweiten Reaktionsstufe das Veresterungsgleichgewicht auf die Esterseite verschoben wird, und damit die Ausbeute an Ester erhöht wird.

Aus einer Destillationskolonne der destillativen Produktreinigung kann man ein Kopfprodukt abziehen, welches aliphatischen Alkohol enthält. Dieser Alkohol lässt sich abtrennen und in das Verfahren zurückführen.

Vorteilhaft ist, dass es das Verfahren erlaubt, Acrylsäure bzw. Methacrylsäure mit bis zu 10 Gew.-%, vorzugsweise bis zu 5 Gew.-%, Diacrylsäure bzw. Dimethacrylsäure als Verunreinigung einzusetzen. Die Diacrylsäure bzw. Dimethacrylsäure wird mit dem erfindungsgemäßen Verfahren zumindest teilweise zum Ester umgesetzt und daher im Gegensatz zu anderen Verfahren nutzbar gemacht.

Als aliphatischen Alkohol, den man in die erste Reaktionsstufe leitet, kann man z. B. Butanol oder 2-Ethylhexanol verwenden.

Ausgestaltungsmöglichkeiten des Verfahrens werden mit Hilfe der Zeichnung erläutert. Es zeigt:
- Fig. 1: ein Fließschema des Verfahrens mit zwei Reaktionsstufen und
- Fig. 2: ein Fließschema mit drei Reaktionsstufen.

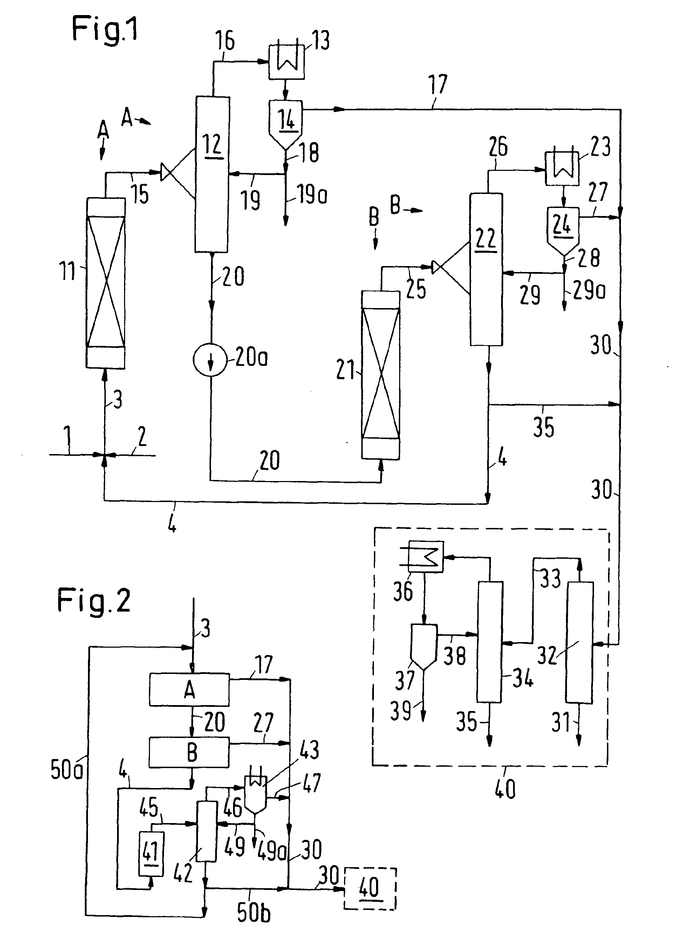

Gemäß Fig. 1 wird in der Leitung (1) Säure, d. h. Acrylsäure oder Methacrylsäure, vorzugsweise zusammen mit einem Polymerisationsinhibitor, herangeführt. Aliphatischer Alkohol kommt aus der Leitung (2). Zum Einsatzgemisch der Leitung (3) gehört auch die in der Leitung (4) zurückgeführte Flüssigkeit. Das Einsatzgemisch tritt in den ersten Veresterungsreaktor (11) ein, der zur ersten Reaktionsstufe (A) gehört. Zu dieser ersten Stufe gehören auch die erste Destillationskolonne (12), der erste Kondensator (13) und der erste Separator (14). Zur zweiten und in diesem Fall letzten Reaktionsstufe (B) gehören der zweite Veresterungsreaktor (21), die zweite Destillationskolonne (22), der zweite Kondensator (23) und der zweite Separator (24). In beiden Reaktoren (11, 21) liegt der Druck im Bereich von 1,5 bis 10 bar und zumeist 2 bis 8 bar.

Aus dem ersten Reaktor (11) zieht man durch die Leitung (15) ein erstes flüssiges Gemisch ab, dessen Estergehalt 20 bis 100 % und vorzugsweise 30 bis 80 % des theoretisch möglichen Esrergehalts beträgt. Dieses Gemisch wird in die erste Kolonne (12) hinein entspannt, wobei der Druck in der ersten Kolonne um mindestens 1 bar niedriger ist als im ersten Reaktor (11). Die gleiche Entspannung der Flüssigkeit der Leitung (25) findet beim Eintritt in die zweite Destillationskolonne (22) statt. Die Temperaturdifferenz zwischen dem Kopf und dem Sumpf der Destillationskolonne (12) beträgt mindestens 10 °C. Das in der Leitung (16) abziehende erste dampfförmige Kopfprodukt enthält Wasserdampf und organische Komponenten, es wird dem Kondensator (13) aufgegeben und praktisch vollständig kondensiert. Das Kondensat gelangt in den Separator (14), aus welchem man durch die Leitung (17) eine organische, esterhaltige Phase abzieht, welche hier als erste organische Phase bezeichnet wird. Für die zweite Reaktionsstufe (B) mit der Destillationskolonne (22), der Leitung (26), dem Kondensator (23) und dem Separator (24) gilt entsprechendes. Hier wird die zweite organische Phase durch die Leitung (27) abgeführt. Die beiden organischen Phasen gelangen in die Sammelleitung (30) und von da in die destillative Produktreinigung (40), die weiter unten erläutert wird.

In der ersten Reaktionsstufe (A) erhält man in der Leitung (18) eine Wasserphase, die hier als "erste Wasserphase" bezeichnet wird. Diese Wasserphase wird durch die Leitung (19) mindestens teilweise in die erste Kolonne zurückgeführt, den Rest entfernt man durch die Leitung (19a) aus dem Verfahren. Man sorgt dafür, dass der Wassergehalt im Sumpfprodukt der Destillationskolonne (12) unter 2 Gew.-% und vorzugsweise unter 1 Gew.-% gehalten wird. Entsprechendes gilt für die Leitungen (28, 29 und 29a) in der zweiten Reaktionsstufe (B).

Die aus der ersten Reaktionsstufe in der Leitung (20) mit der Pumpe (20a) abgezogene Flüssigkeit, die hier als erstes Sumpfprodukt bezeichnet wird, welche Ester, Alkohol und Säure enthält, wird in die zweite Reaktionsstufe und hier in den zweiten Reaktor (21) geführt. Das Sumpfprodukt der zweiten Destillationskolonne (22) enthält Diacrylsäure oder Dimethacrylsäure, wobei der Anteil der Säure im Sumpfprodukt 1 bis 30 Gew.-% beträgt. Sollte es eine dritte Reaktionsstufe geben, wird das Sumpfprodukt der zweiten Destillationskolonne (22) mindestens teilweise in diese dritte Stufe geführt. Im vorliegenden Fall aber teilt man das Sumpfprodukt auf und gibt einen ersten Teilstrom durch die Leitung (4a) in die Sammelleitung (30) und man führt den Rest durch die Leitung (4) zurück in die erste Reaktionsstufe.

Die destillative Produktreinigung (40) arbeitet im vorliegenden Fall folgendermaßen:

Die esterhaltige Flüssigkeit der Leitung (30) gelangt in eine Destillationskolonne (32), aus deren Sumpf man das gewünschte Esterprodukt durch die Leitung (31) abzieht. Das alkoholhaltige Kopfprodukt strömt durch die Leitung (33) zu einer weiteren Destillationskolonne (34), aus deren Sumpf man durch die Leitung (35) aliphatischen Alkohol abzieht. Das Kopfprodukt, im wesentlichen Wasser, kühlt man im Kondensator (36), führt das Kondensat zu einem Separator (37) und leitet eine alkoholreiche organische Phase durch die Leitung (38) zurück in die Kolonne (34). Wasser wird durch die Leitung (39) aus dem Verfahren entfernt. Den in der Leitung (35) anfallenden Alkohol kann man im Verfahren wieder verwenden.

Fig. 2 zeigt ein dreistufiges Verfahren, wobei sich die dritte Reaktionsstufe mit den Teilen mit Bezugsziffem (41) bis (50b) an die Stufen (A) und (B) anschließt. Zur dritten Stufe gehören der Veresterungsreaktor (41), die Destillationskolonne (42), der vereinfacht dargestellte Kondensator- und Separatorteil (43) und die Rückführung (50a) für Sumpfprodukt. Die Sammelleitung (30) führt Kopfprodukte und einen Teilstrom (50b) des Sumpfprodukts in die bereits zu Fig. 1 erläuterte destillative Produktreinigung (40). Durch die Reaktionsstufen kann man die Ausbeute am Ester-Produkt erhöhen.

### Beispiel 1:

In einer etwa der Fig. 1 der Zeichnung entsprechenden Labor-Anordnungen wird in zwei Reaktionsstufen Acrylsäure (AA) mit 1-Butanol (BuOH) verestert. Man verwendet aus Edelstahl bestehende, ummantelte zylindrische Reaktoren (11) und (21), die mittels Wärmeträgeröl auf 110 °C beheizt werden. Beide Reaktoren sind mit jeweils 400 ml des aciden Ionenaustauscher-Katalysators Bayer K 1221 gefüllt. Dem ersten Reaktor (11) ist eine Destiliationskolonne (12) aus Glas (Doppelmantel, beheizt mit Wärmeträgeröl auf 130 °C, gefüllt mit Drahtwendeln als Füllkörper) nachgeschaltet. Entsprechend einer Raumgeschwindigkeit LHSV von 1,0 l/(l•h) wird der erste Reaktor (11) mit 400 ml/h flüssigem Einsatzstoff folgender Zusammensetzung durch die Leitung (3) beaufschlagt: Acrylsäure (AA) 41,7 Gew.-%; 1-Butanol (BuOH) 54,7 Gew.-%; Diacrylsäure (DiAA als Verunreinigung der AA) 2,6 Gew.-%; p-Methoxyphenol (PMP) als Polymerisationsinhibitor 1 Gew.-%. Die Reaktoraustrittsdrücke werden mittels Druckhalteventilen auf 4 bar eingestellt. Der den ersten Reaktor verlassende Produktstrom (15) wird in die Destillationskolonne (12) hinein entspannt, die bei Atmosphärendruck betrieben wird. Das als Kopfprodukt der Destillation anfallende, ternäre Azeotrop aus Butylacrylat (BA), BuOH und Wasser geht bei 90 °C über, wird mittels Kondensator (13) gekühlt und einem Separator (14) zugeführt. Die sich abscheidenden organischen (17) und wässrigen Phasen (18) werden gesammelt und gewogen, stündlich werden dabei 42 g der organischen Phase und 20 g der wässrigen Phase gesammelt. Das Sumpfprodukt (20) der Destillation, 300 g/h, wird in einem Zwischengefäß gesammelt und anschließend mittels einer Kolbenpumpe (20a) dem zweiten Veresterungsreaktor (21) zugeführt. Der den zweiten Veresterungsreaktor verlassende Produktstrom (25) wird gesammelt und zur Kontrolle gewogen. Für die gaschromatographische und nasschemische Analyse werden den Leitungen (15), (17), (18), (20) und (25) Proben entnommen. Die Ergebnisse der Analysen sind in der nachfolgenden Tabelle 1 zusammengestellt (Angaben in Gew.-%), wobei Nebenbestandteile nicht berücksichtigt sind:

| **Leitung** | **(15)** | **(20)** | **(17)** | **(18)** | **(25)** |
|---|---|---|---|---|---|
| **AA** | 16,9 | 19,3 | 0,1 | 0,1 | 8,8 |
| **BuOH** | 20,4 | 16,4 | 49,6 | 6,6 | 10,5 |
| **BA** | 46,4 | 49,6 | 41,1 | 0,0 | 61,3 |
| **H₂O** | 6,4 | 0,0 | 9,2 | 93,3 | 1,8 |

Der den zweiten Veresterungsreaktor verlassende Produktstrom (25) ist weitgehend frei von Dibutylether (< 0,1 Gew.-%). Der AA-Umsatz beträgt 60,0 % im ersten Veresterungsreaktor (11), der AA-Gesamtumsatz nach zwei Reaktordurchgängen mit zwischengeschalteter Destillation (12) liegt bei 79,0 %. Die BA-Ausbeute, bezogen auf eingesetzte AA, beträgt nach dem ersten Veresterungsreaktor (11) 61,9 %, nach zwei Reaktordurchgängen mit zwischengeschalteter Destillation (12) 82,2 % (BA im Kopfprodukt der Destillation nicht berücksichtigt). Damit ergeben sich formal BA-Selektivitäten > 100 %, wenn man nur AA berücksichtigt. Dies ist auf die Umsetzung der im Einsatzstoff enthaltenen Diacrylsäure zurückzuführen. Der den zweiten Veresterungsreaktor (21) verlassende Produktstrom (25) enthält lediglich noch 0,37 Gew.-% DiAA, woraus sich ein DiAA-Gesamtumsatz von 85,9 % ergibt.

Ein Ausführungsbeispiel, das drei Reaktionsstufen umfasst und von Beispiel 1 ausgeht, wird im nachfolgenden **Beispiel 2** beschrieben.

### Beispiel 2:

Im Labor wird gemäß Fig. 2 gearbeitet, wobei die Daten teilweise berechnet sind. Alle drei Veresterungsreaktoren werden bei 110 °C und 4 bar betrieben. Die Raumgeschwindigkeit wird so gewählt, dass der AA-Umsatz in den 3 Reaktoren jeweils 60 % beträgt. Polymerisationsinhibitor wird den Leitungen (3), (17), (27) und (47) zugegeben. Die Destillation (42) wird unter den in Beispiel 1 beschriebenen Bedingungen betrieben. Die destillative Produktreinigung (40) arbeitet wie zusammen mit Fig. 1 beschrieben.

Die Stoffmengenströme der Hauptbestandteile der dreistufigen Veresterung von 1,825 mol/h Acrylsäure mit 2,373 mol/h 1-Butanol zeigt Tabelle 2. Die Stoffmenge in der Leitung (3) umfasst bereits den Rücklaufstrom (50a).

**Tabelle 2**

| **Leitung** | **AA (mol/h)** | **BuOH (mol/h)** | **BA (mol/h)** | **H₂O (mol/h)** |
|---|---|---|---|---|
| 3 | 1,9062 | 2,6000 | 1,9312 | 0,1514 |
| 15 | 0,7623 | 1,4568 | 3,0751 | 1,247 |
| 17 | 0,0026 | 0,3634 | 0,6005 | 0,4087 |
| 19a | 0,0002 | 0,0038 | 0,0006 | 0,7314 |
| 19 | 0,0006 | 0,0153 | 0,0025 | 2,8966 |
| 20 | 0,7594 | 1,0891 | 2,4736 | 0,1265 |
| 25 | 0,3038 | 0,6335 | 2,9297 | 0,5824 |
| 27 | 0,0075 | 0,0883 | 0,2074 | 0,1235 |
| 29a | 0,0006 | 0,0010 | 0,0002 | 0,2203 |
| 29 | 0,0025 | 0,0040 | 0,0009 | 0,8733 |
| 4 | 0,2957 | 0,5443 | 2,7222 | 0,2309 |
| 45 | 0,1183 | 0,3669 | 2,8997 | 0,4081 |
| 47 | 0,0027 | 0,0408 | 0,1385 | 0,0622 |
| 49a | 0,0003 | 0,0005 | 0,0001 | 0,1314 |
| 49 | 0,0010 | 0,0018 | 0,0005 | 0,5268 |
| 50 | 0,1153 | 0,3256 | 2,7615 | 0,2167 |
| 50a | 0,0806 | 0,2273 | 1,9317 | 0,1514 |
| 50b | 0,0346 | 0,0975 | 0,8281 | 0,0649 |
| 30 | 0,0473 | 0,5899 | 1,7744 | 06593 |
| 33 | 0,0000 | 0,5739 | 0,0068 | 0,6594 |
| **31** | **0,0473** | **0,0164** | **1,7673** | **0,0000** |
| 39 | 0,0000 | 0,0067 | 0,0001 | 0,5842 |
| 38 | 0,0000 | 0,1545 | 0,0099 | 0,2469 |
| 35 | 0,0000 | 0,5667 | 0,0067 | 0,0751 |

Die Destillationskolonne (32) wird bei 1 bar betrieben, das Produkt der Leitung (31) besteht zu 98 Gew.-% aus Butylacrylat. Die auf eingesetzte Acrylsäure bezogene Butylacrylat-Ausbeute beträgt 97 %. Das Sumpfprodukt, das in der Leitung (35) anfällt, kann man dem Gemisch der Leitung (3) zugeben, was aber hier nicht berücksichtigt ist. Falls Acrylsäure mit einem hohen Anteil an Diacrylsäure als Einsatzstoff verwendet wird oder eine besonders hohe BA-Reinheit erforderlich ist, empfiehlt sich die Produktabnahme über einen Seitenstrom im unteren Drittel der Destillationskolonne (32).

## Patentansprüche

1. Verfahren zur Herstellung eines Esters der Acrylsäure oder Methacrylsäure durch Umsetzung mit einem aliphatischen Alkohol mit 1 bis 10 C-Atomen in Gegenwart eines Katalysators in mindestens 2 und höchstens 5 in Serie geschalteten jeweils aus einem Veresterungsreaktor mit anschließender Destillationskolonne zum Trennen des von dem Veresterungsreaktor kommenden flüssigen Produkts bestehenden Reaktionsstufen, indem
1. Acrylsäure oder Methacrylsäure zusammen mit dem aliphatischen Alkohol durch den einen Druck von 1,5 bis 10 bar aufweisenden Veresterungsreaktor der ersten Reaktionsstufe geleitet wird,
2. aus dem Veresterungsreaktor der ersten Reaktionsstufe ein Ester mit einem Gehalt von 20 bis 100 % des theoretisch möglichen Estergehalts, sowie Säure, Alkohol und Wasser enthaltendes flüssiges Gemisch abgezogen und in die nachgeschaltete, einen um mindestens 1 bar niedrigeren Druck als im Veresterungsreaktor aufweisende Destillationskolonne entspannt wird,
3. aus der Destillationskolonne der ersten Reaktionsstufe ein dampfförmiges, Wasserdampf und organische Komponenten enthaltendes Kopfprodukt abgezogen, gekühlt, kondensiert und das Kondensat in einem Separator in eine organische Phase und in eine alkoholhaltige Wasserphase getrennt wird,
4. aus dem einen Druck von 1,5 bis 10 bar aufweisenden Veresterungsreaktor der zweiten Reaktionsstufe ein Ester, Säure, Alkohol und Wasser enthaltendes flüssiges Gemisch abgezogen und in die anschließende, einen um mindestens 1 bar als im Veresterungsreaktor aufweisende Destillationskolonne entspannt wird,
5. aus der Destillationskolonne der zweiten Reaktionsstufe ein dampfförmiges Wasserdampf und organische Komponenten enthaltendes Kopfprodukt abgezogen, gekühlt, kondensiert und das Kondensat in einem Separator in eine organische Phase und in eine Wasserphase getrennt wird,
**dadurch gekennzeichnet, dass**
6. die Veresterungsreaktoren der Reaktionsstufen jeweils mit einem Festbett aus acidem Ionenaustauscher-Katalysator gefüllt sind,
7. aus dem Sumpf der Destillationskolonne der ersten bis zweitletzen Reaktionsstufe ein Ester, Alkohol und Säure enthaltendes Produkt abgezogen und mindestens teilweise in den Veresterungsreaktor der folgenden Reaktionsstufe geleitet wird,
8. die aus dem Kopfprodukt der Destillationskolonne der Reaktionsstufen abgetrennte Wasserphase mindestens teilweise in die jeweilige Destillationskolonne zurückgeführt wird,
9. die aus dem Kopfprodukt der Destillationskolonne der Reaktionsstufen abgetrennte organische esterhaltige Phase mindestens teilweise einer destillativen Produktreinigung zugeführt wird,
10. das aus der Destillationskolonne der letzten Reaktionsstufe abgezogene Sumpfprodukt in den Veresterungsreaktor der vorhergehenden Reaktionsstufe geleitet wird und
11. aus der destillativen Reinigung Ester abgezogen wird.

2. Verfahren nach Anspruch 1, **dadurch gekennzeichnet, dass** man einen Teil des Sumpfprodukts der Destillationskolonne der letzten Reaktionsstufe in die destillative Produktreinigung leitet.

3. Verfahren nach Anspruch 1 oder 2, **dadurch gekennzeichnet, dass** im aus der ersten oder zweiten Kolonne abgezogenen Sumpfprodukt Acrylsäure oder Methacrylsäure enthalten ist, wobei der Anteil der Acrylsäure oder der Methacrylsäure im Sumpfprodukt 1 bis 30 Gew.-% beträgt, und der Wasseranteil im Sumpfprodukt unter 2 Gew.-% beträgt.

4. Verfahren nach Anspruch 1 oder einem der folgenden, **dadurch gekennzeichnet, dass** das aus der Destillationskolonne der ersten Reaktionsstufe abgezogene erste Sumpfprodukt einen Wassergehalt von höchstens 1 Gew.-% aufweist.

5. Verfahren nach Anspruch 1 oder einem der folgenden, **dadurch gekennzeichnet, dass** man aus einer Destillationskolonne der destillativen Produktreinigung ein Kopfprodukt abzieht, aus welchem man aliphatischen Alkohol abtrennt.

6. Verfahren nach einem der Ansprüche 1 bis 5, **dadurch gekennzeichnet, dass** man als aliphatischen Alkohol Butanol oder 2-Ethylhexanol in die erste Reaktionsstufe leitet.

7. Verfahren nach Anspruch 1 oder einem der folgenden, **dadurch gekennzeichnet, dass** man mit drei oder vier Reaktionsstufen arbeitet.

## Claims

1. A process for producing an ester of acrylic acid or methacrylic acid by reaction with an aliphatic alcohol having 1 to 10 C atoms in the presence of a catalyst in at least 2 and not more than 5 series-connected reaction stages, which each consist of an esterification reactor with downstream distillation column for separating the liquid product coming from the esterification reactor, in that
1. acrylic acid or methacrylic acid together with the aliphatic alcohol is passed through an esterification reactor of the first reacction stage, which has a pressure of 1.5 to 10 bar,
2. from the esterification reactor of the first reaction stage, an ester with a content of 20 to 100% of the theoretically possible ester content and a liquid mixture containing acid, alcohol and water is withdrawn and discharged into the downstream distillation column having a pressure lower by at least 1 bar than in the esterification reactor,
3. from the distillation column of the first reaction stage, a vaporous top product containing steam and organic components is withdrawn, cooled and condensed, and in a separator the condensate is separated into an organic phase and an alcohol-containing aqueous phase,
4. from the esterification reactor of the second reaction stage, which has a pressure of 1.5 to 10 bar, a liquid mixture containing ester, acid, alcohol and water is withdrawn and discharged into the downstream distillation column having a pressure lower by at least 1 bar than in the esterification reactor,
5. from the distillation column of the second reaction stage, a vaporous top product containing steam and organic components is withrawn, cooled and condensed, and in a separator the condensate is separated into an organic phase and an aqueous phase,
**characterized in that**
6. the esterification reactors of the reaction stages each are filled with a fixed bed of an acidic ion exchanger catalyst,
7. from the bottom of the distillation column of the first to last reaction stages but one, a product containing ester, alcohol and acid is withdrawn and is at least partly introduced into the esterification reactor of the following reaction stage,
8. the aqueous phase separated from the top product of the distillation column of the reaction stages is at least partly recirculated to the respective distillation column,
9. the organic ester-containing phase separated from the top product of the distillation column of the reaction stages is at least partly supplied to a distillative product cleaning,
10. the bottom product withdrawn from the distillation column of the last reaction stage is introduced into the esterification reactor of the preceding reaction stage, and
11. ester is withdrawn from the distillative cleaning.

2. The process according to claim 1, **characterized in that** part of the bottom product of the distillation column of the last reaction stage is introduced into the distillative product cleaning.

3. The process according to claim 1 or 2, **characterized in that** the bottom product withdrawn from the first or second column contains acrylic acid or methacrylic acid, wherein the content of acrylic acid or methacrylic acid in the bottom product is 1 to 30 wt-%, and the water content in the bottom product is below 2 wt-%.

4. The process according to claim 1 or any of the preceding claims, **characterized in that** the first bottom product withdrawn from the distillation column of the first reaction stage has a water content of not more than 1 wt-%.

5. The process according to claim 1 or any of the preceding claims, **characterized in that** from a distillation column of the distillative product cleaning a top product is withdrawn, from which aliphatic alcohol is separated.

6. The process according to any of claims 1 to 5, **characterized in that** butanol or 2-ethylhexanol is introduced into the first reaction stage as aliphatic alcohol.

7. The process according to claim 1 or any of the preceding claims, **characterized in that** three or four reaction stages are employed.

## Revendications

1. Procédé de préparation d'un ester de l'acide acrylique ou de l'acide méthacrylique par réaction sur un alcool aliphatique ayant de 1 à 10 atomes de carbone en présence d'un catalyseur dans au moins 2 et au plus 5 étages de réaction montés en série et constitués, respectivement, d'un réacteur d'estérification ayant une colonne de distillation raccordée pour la séparation du produit liquide venant du réacteur d'estérification, dans lequel
1. on fait passer de l'acide acrylique ou de l'acide méthacrylique ensemble avec l'alcool aliphatique dans un réacteur d'estérification ayant une pression de 1,5 à 10 bar du premier étage de réaction,
2. on soutire du réacteur d'estérification du premier étage de réaction un mélange liquide contenant un ester ayant une teneur représentant de 20 à 100 % de la teneur en ester possible théoriquement ainsi que de l'acide, de l'alcool et de l'eau et on le détend dans la colonne de distillation en aval ayant une pression plus basse d'au moins un bar que dans le réacteur d'estérification,
3. on soutire de la colonne de distillation du premier étage de réaction un produit de tête sous forme de vapeur contenant de la vapeur d'eau et des constituants organiques, on le condense et on sépare le produit condensé dans un séparateur en une phase organique et en une phase aqueuse contenant de l'alcool,
4. on soutire du deuxième réacteur d'estérification du deuxième étage de réaction ayant une pression de 1,5 à 10 bar un mélange liquide contenant un ester, de l'acide, de l'alcool et de l'eau et on le détend dans la colonne de distillation raccordée ayant une pression plus basse d'au moins 1 bar que dans le réacteur d'estérification,
5. on soutire de la colonne de distillation du deuxième étage de réaction un produit de tête sous forme de vapeur et contenant de la vapeur d'eau et des constituants organiques, on le refroidit et on le condense et on sépare le produit condensé dans un séparateur en une phase organique et en une phase aqueuse,
**caractérisé en ce que**
6. on garnit les réacteurs d'estérification des étages de réaction respectivement d'un lit fixe constitué de catalyseur échangeur d'ion acide,
7. on soutire du bas de la colonne de distillation des premier à avant-dernier étages de réaction un produit contenant de l'ester, de l'alcool et de l'acide et on l'envoie, au moins en partie, dans le réacteur d'estérification des étages de réaction suivants,
8. on retourne , au moins en partie , dans la colonne de distillation respective la phase aqueuse séparée du produit de tête de la colonne de distillation des étages de réaction,
9. on envoie, au moins en partie, à une purification du produit par distillation la phase organique contenant de l'ester séparée du produit de tête de la colonne de distillation des étages de réaction,
10. on envoie dans le réacteur d'estérification de l'étage de réaction précédent le produit de queue soutiré de la colonne de distillation du dernier étage de réaction et
11. on soutire de l'ester de la purification par distillation.

2. Procédé suivant la revendication 1, **caractérisé en ce que** l'on envoie une partie du produit de queue de la colonne de distillation du dernier étage de réaction à la purification du produit par distillation.

3. Procédé suivant la revendication 1 ou 2, **caractérisé en ce que** dans le produit de queue soutiré de la première ou de la deuxième colonne est contenu de l'acide acrylique et de l'acide méthacrylique, la proportion de l'acide acrylique et de l'acide méthacrylique dans le produit de queue représentant de 1 à 30 % en poids et la proportion d'eau dans le produit de queue étant inférieure à 2 % en poids.

4. Procédé suivant la revendication 1 ou l'une des suivantes, **caractérisé en ce que** le premier produit de queue soutiré de la colonne de distillation du premier étage de réaction a une teneur en eau d'au plus 1 % en poids.

5. Procédé suivant la revendication 1 ou l'une des suivantes, **caractérisé en ce que** l'on soutire d'une colonne de distillation de la purification du produit par distillation un produit de tête dont on sépare l'alcool aliphatique.

6. Procédé suivant l'une des revendications 1 à 5, **caractérisé en ce que** l'on envoie dans le premier étage de réaction comme alcool aliphatique du butanol ou du 2-éthylhexanol.

7. Procédé suivant la revendication 1 ou l'une des suivantes, **caractérisé en ce que** l'on travaille avec trois ou quatre étages de réaction.
